# EUROPEAN PATENT SPECIFICATION

(11) **EP 2 785 288 B1**
(45) Date of publication and mention of the grant of the patent: **10.05.2017**
(21) Application number: 12854597.7
(22) Date of filing: 04.12.2012
(51) Int. Cl.: A61B 17/3207, A61B 17/22, A61M 25/00, A61B 5/026, A61B 18/24, A61M 25/10

(54) **A LESION TREATMENT DEVICE**
VORRICHTUNG ZUR BEHANDLUNG VON LÄSIONEN
DISPOSITIF DE TRAITEMENT DE LÉSIONS

(30) Priority: 04.12.2011 US 201161566667 P
(43) Date of publication of application: 08.10.2014
(73) Proprietor: Asymmetric Medical Ltd., 76854 Kfar Mordecai (IL)
(72) Inventor: ESHKOL, Moshe, 60917 Harutzim (IL); WEISBERG, Ori, 76855 Shdema (IL)
(74) Representative: Pearl Cohen Zedek Latzer Baratz UK LLP
(86) International application number: PCT/IL2012/050496
(87) International publication number: WO 2013/084227

(56) References cited:
- EP-A1- 0 519 063
- EP-A1- 2 367 482
- WO-A1-2011/157362
- WO-A1-2013/114376
- WO-A2-2005/120628
- WO-A2-2005/120628
- DE-A1- 19 846 663
- US-A- 5 487 740
- US-A1- 2011 077 663
- US-A1- 2011 251 582
- US-A1- 2011 251 582

## Description

### BACKGROUND

### 1. TECHNICAL FIELD

The present invention relates to the field of treating flow obstructions or protrusions in vessels and/or passages in biological systems, and more particularly, to cardiovascular lesion treatment.

### 2. DISCUSSION OF RELATED ART

Atherosclerosis, which is a major cause of cardiovascular disease resulting in myocardial infarction, stroke and other major medical complications, is characterized by the progressive accumulation of atherosclerotic deposits (known as plaque) on the inner walls of arteries. Consequently, blood flow is obstructed and there is increased likelihood of clot formation that can partially or completely block or occlude an artery. Even in cases where occlusion doesn't occur, plaque is a risk factor as certain non-stable deposits, known as vulnerable plaque, may dislodge and cause a stroke or myocardial infarction. Arteries narrowed as a result of atherosclerosis that cannot be treated effectively by drug therapy are treated by medical procedures designed to restore blood flow, including highly invasive procedures such as coronary artery bypass surgery and less invasive procedures such as balloon angioplasty, atherectomy and stenting.

Bypass surgery involves opening the patient's chest and transferring a vein cut from the patient's leg to the heart to construct a detour around the occluded artery. Bypass surgery requires prolonged hospitalization and an extensive recuperation period. Furthermore, bypass surgery also exposes the patient to a risk of major surgical complications. Balloon angioplasty is a less invasive and costly alternative to bypass surgery and is performed in a hospital cardiac catheterization laboratory by an interventional cardiologist. In this procedure, a balloon-tipped catheter is inserted into a blood vessel through a small incision in the patient's arm or leg. The physician uses a guide catheter to feed the balloon through the patient's blood vessels to the occluded artery. At that point, a guidewire is inserted across the deposits of atherosclerotic plaque, known as lesions, to provide a pathway for the balloon catheter. The deflated balloon is advanced over the guidewire, positioned within the occluded area and inflated and deflated several times. This inflation and deflation usually tears the plaque and expands the artery beyond its point of elastic recoil. Thus, although no plaque is removed the opening through which the blood flows is enlarged.

Atherectomy employs a rotating mechanical device mounted on a catheter to cut and remove plaque from a diseased artery.

Another approach to treating atherosclerosis or thrombosis is to degrade thrombi and remove plaque using various pharmacologic agents. Many techniques currently exist to deliver medicaments and other active agents to body tissues. These include oral administration, direct injection to the tissue and intravenous administration. These mechanisms are systemic in that they deliver the active agent via the bloodstream throughout the entire body. Effective pharmacologic or drug therapy requires achieving adequate concentrations of the active drug at the desired treatment site without producing drug concentrations elsewhere in the body that may cause undesirable and even dangerous side effects.

Laser angioplasty removes plaques using light in varying wavelengths ranging from ultraviolet to infrared that is delivered to the lesion by a fiber-optic catheter. Early attempts to develop a laser angioplasty system used continuous wave thermal lasers that generated heat to vaporize plaque. These laser systems caused charring and significant thermal damage to healthy tissue surrounding the lesion. As a result, thermal laser systems have generally been regarded as inappropriate for use in coronary arteries. Conversely, excimer lasers use ultraviolet light to break the molecular bonds of the atherosclerotic plaque, a process known as photoablation. Excimer lasers use electrically excited xenon and chloride gases to generate an ultraviolet laser pulse with a wavelength of 308nm. This UV light wavelength is absorbed by the proteins and lipids that comprise plaque, resulting in precise plaque disintegration and thus, blood flow restoration without significant thermal damage to surrounding tissue. The ablated plaque is converted into carbon dioxide and other gases, as well and minute particulate matter that can be easily eliminated. Similarly, Ultrasound and RF are sometimes used for the ablation of plaque.

US 2011251582 discloses medical devices that are expandable from a compressed state to an expanded state. Wherein, the medical devices are configured such that when the substrate is in a compressed state, its sheath is also, and a perforation therein is closed. When the substrate is in an expanded state, its sheath is also, and the perforations are open, thereby allowing release of a treatment agent. The release of a drug delivers is mechanical, and requires activation by the user. The challenge remains to detect and treat vascular lesions in an effective and safe manner.

### BRIEF SUMMARY

One aspect of the present invention provides a device for applying a treatment to an obstruction, comprising a flexible treatment layer that comprises a plurality of operable elements configured to be mechanically activated to apply the treatment to the obstruction upon bending of the flexible treatment layer beyond a specified curvature threshold due to contact thereof with the obstruction and to be mechanically de-activated upon a specified decrease of the bending, resulting from the treatment of the obstruction. These, additional, and/or other aspects and/or advantages of the present invention are: set forth in the detailed description which follows; possibly inferable from the detailed description; and/or learnable by practice of the present invention. The scope of the invention is defined by the appended claims.

### BRIEF DESCRIPTION OF THE DRAWINGS

For a better understanding of embodiments of the invention and to show how the same may be carried into effect, reference will now be made, purely by way of example, to the accompanying drawings in which like numerals designate corresponding elements or sections throughout.

In the accompanying drawings:
**Figures 1A-1C** are high level schematic illustrations of a cross section of a vessel with an obstruction, contacted by a device for applying a treatment to the obstruction, according to some embodiments of the invention;
**Figures 2A- 2D** are high level schematic illustrations of a partial longitudinal section of a vessel with a lesion, contacted by a device for applying a treatment to an obstruction such as a vascular lesion, according to some embodiments of the invention;
**Figures 3A** and **3B** are high level schematic illustrations of a device configuration having a non-active state and a bending-activated state, respectively, according to some embodiments of the invention;
**Figures 4A** and **4B** are high level schematic illustrations of a device for perforating the lesion, having a non-active state and a bending-activated state, respectively, according to some embodiments of the invention;
**Figures 5A** and **5B** are high level schematic illustrations of a device for perforating the lesion, having a non-active state and a bending-activated state, respectively, and including an actuator, according to some embodiments of the invention;
**Figures 6A and 6B** are high level schematic illustrations of a device for delivering a drug to the lesion, having a non-active state and a bending-activated state, respectively, according to some embodiments of the invention;
**Figures 7A** and **7B** are high level schematic illustrations of a device for delivering a drug to the lesion, having a non-active state and a bending-activated state, respectively, according to some embodiments of the invention;
**Figure 8A** and **8B** are high level schematic illustrations of a device having a flexible network with varying mesh openings, according to some embodiments of the invention;
**Figures 9A** and **9B** are high level schematic illustrations of a device for delivering a drug to the lesion, according to some embodiments of the invention;
**Figure 10** is a high level schematic illustration of a balloon implemented device for treating lesions at vessel bifurcations, according to some embodiments of the invention;
**Figures 11A** and **11B** are high level schematic illustrations of a device for focusing energy at the lesion, having a non-active state and a bending-activated state, respectively, according to some embodiments of the invention;
**Figures 12A-12C** are high level schematic illustrations of a parachute implemented device in stages of operation, according to some embodiments of the invention;
**Figures 13A** and **13B** are high level schematic illustrations of an obstruction grinding device, according to some embodiments of the invention;
**Figures 14A-14E** are high level schematic illustrations of a one layered treatment device, according to some embodiments of the invention;
**Figures 15A-15C** are high level schematic illustrations of openings as operable elements, according to some embodiments of the invention; and
**Figure 16** is a high level flowchart illustrating an exemplary method of treating an obstruction in a vessel.

### DETAILED DESCRIPTION

Prior to setting forth the detailed description, it may be helpful to set forth definitions of certain terms that will be used hereinafter.

The term "vessel" as used herein in this application refers to any hollow container or pipe for moving fluids. For example, the term "vessel" refers to a blood vessel, especially an artery, parts of the gastrointestinal tract, fallopian tubes, urinary or bile tracts, airways and bronchi and other bodily vessels, as well as to pipes and tubes for industrial uses.

The term "obstruction" as used herein in this application refers to any piece of matter that retards flow through a vessel. In particular, the term "obstruction" as used herein in this application includes lesions, or vascular lesions, which are deposits on arteries (e.g. plaque) as well as other distractions to normal blood flow. The terms "obstruction" and "lesion" are used interchangeably throughout the specifications, in a non-limiting manner.

With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. The scope of the invention is defined by the claims appended hereto. In this regard, no attempt is made to show structural details of the invention in more detail than is necessary for a fundamental understanding of the invention, the description taken with the drawings making apparent to those skilled in the art how the several forms of the invention may be embodied in practice.

Before explaining at least one embodiment of the invention in detail, it is to be understood that the invention is not limited in its application to the details of construction and the arrangement of the components set forth in the following description or illustrated in the drawings. The invention is applicable to other embodiments or of being practiced or carried out in various ways. Also, it is to be understood that the phraseology and terminology employed herein is for the purpose of description and should not be regarded as limiting.

The present invention enables removing and/or treating obstructions in vessels and in some cases simultaneously delivering pharmacologic therapy to a selected site in the body lumen in a simple way. The invention may treat any type of obstruction in any type of vessel, combines an automatic mechanical obstruction identification mechanism with a treatment procedure to remove and/or treat the obstruction. In an example of the obstruction being a vascular lesion, the invention may be used to remove plaque without generating embolic material which can create a risk of ischemic stroke. Furthermore, the devices described by this invention automatically identify and restrict treatment to the sclerotic area, leaving adjacent tissue unharmed.

Among other, this disclosure relates to exemplary methods and apparatuses for excising and/or treating mobile and non-mobile atheromas. The device includes an automated lesion identification mechanism and a treatment mechanism. In general both automated lesion detection unit and the treatment head may be inserted into the body via a catheter which may operate in different regions of the human body. For example, the aorta, common carotid artery, external and internal carotid arteries, brachiocephalic trunk, middle cerebral artery, anterior cerebral artery, posterior cerebral artery, vertebral artery, basilar artery, subclavian artery, brachial artery, axillary artery, Iliac artery, renal artery, femoral artery, popliteal artery, celiac artery, superior mesenteric artery, inferior mesenteric artery, anterior tibial artery, posterior tibial artery, the coronary arteries and all other arteries. The catheter may optionally include a blood filter means which enable to capture plaque inadvertently dislodged during an atherectomy procedure.

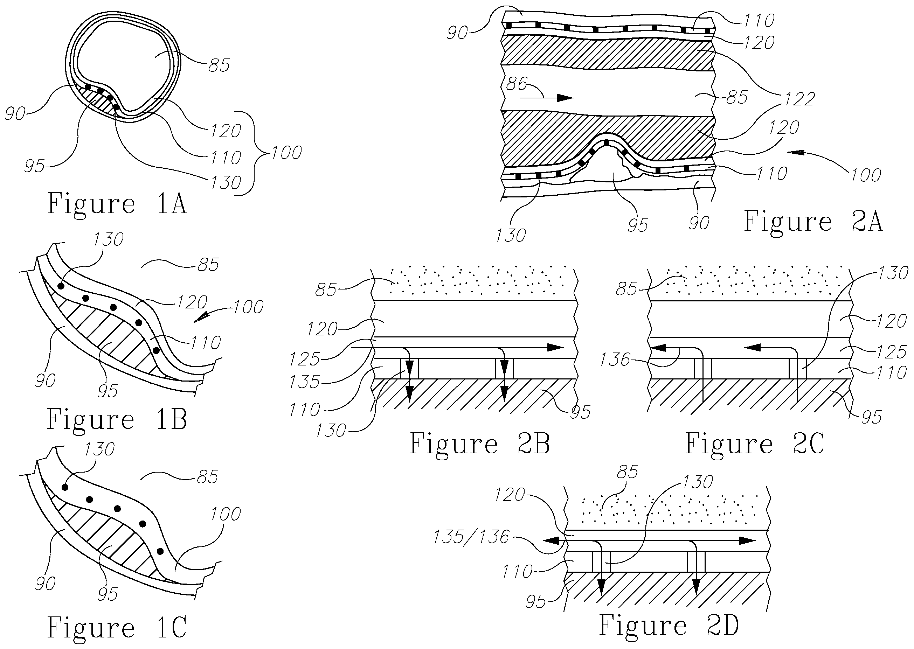

**Figures 1A-1C** are high level schematic illustrations of a cross section of a vessel **90** with an obstruction **95**, contacted by a device **100** for applying a treatment to obstruction **95**, according to some embodiments of the invention. **Figures 2A-2D** are high level schematic illustrations of a partial longitudinal section of vessel **90** contacted by device **100** for applying a treatment to obstruction **95** such as a vascular lesion, according to some embodiments of the invention.

Device **100** comprises a flexible supporting layer **120** and a treatment layer **110** comprising a plurality of operable elements **130** attached to flexible supporting layer **120** and configured to be mechanically activated to apply the treatment to obstruction **95** upon bending of flexible supporting layer **120** due to its contact with obstruction **95**. For example, operable elements **130** may be mechanically activated upon bending of flexible supporting layer **120** beyond a specified curvature threshold, as illustrated in examples below. Generally, a smaller curvature radius causes a stronger bending which activated device **100**. Upon reduction if the curvature radius, de-activation may commence when the curvature radius is larger than the threshold. Hence the expression "beyond a specified curvature threshold" in the application should be interpreted as relating to a larger curvature and a smaller curvature radius.

The bending of device **100** may result from its contact with obstruction **95** or by active pressing of device **100** upon obstruction **95** to activate operable elements **130**.

In embodiments, device **100** comprises a single layer having operable elements **130** such as openings going through the whole thickness of device **100**. Such embodiment is illustrated in **Figures 1C**, **14A-E** and **15A-C**. In embodiments, the material of device **100** itself may be used to treat obstruction **95**.

In embodiments, operable elements **130** are further configured to be mechanically de-activated upon a specified decrease of the bending, due to the treatment of obstruction **95**. For example, after the size of obstruction **95** decreases due to the treatment, the treatment may be mechanically interrupted as the curvature of device **100** decreases to a certain degree, e.g. below the specified curvature threshold or below a different threshold, determined according to the structure of the surrounding tissue.

As illustrated in **Figure 1A** and **1B**, device **100** is configured to bend upon contact with obstruction **95**. The bending causes a mechanical activation of operable elements **130**, as illustrated below. In some embodiments, operable elements **130** may comprise or be sensors. Device **100** may be hollow, and enable blood flow through cavity **85** in device **100**.

The following are some non-limiting examples for curvature thresholds with respect to various obstructions. It should be noted, as illustrated below, that generally there are two dimensions related to the curvature radius. One is in the radius of vessel **90** (in a plane perpendicular to the vessel) and the other is related to the longitudinal way along vessel **90** (in a longitudinal cross section of vessel **90**). As a result, operable elements **130** (such as openings **114**) may be asymmetric and have different dimensions in different directions, or be oriented in different ways along device **100** (and with respect to vessel **90**). Curvature thresholds may differ in different directions, as in the longitudinal axis the reference radius (that of vessel **90** without any obstruction **95**) is very large, while in the cross sectional axis the reference radius (without obstruction **95**) is much smaller. Hence, different activation and de-activation thresholds may be defined for these directions and may be used to apply coarser of finer treatments, possibly simultaneously in different regions or sequentially and complementary in one region of obstruction **95**.

In non limiting examples, the following may be typical parameters for the curvature threshold in cases of different obstructions. In case of coronary lesions having a radius between 0.1 mm and 2 mm, the specified curvature threshold may be about two times the lesion radius, or up to five times the lesion radius to achieve a more thorough removal of the lesion. Device **100** may be adapted or selected according to the specific lesion that is to be treated therewith.

In case of vascular lesions having a radius between 1 mm and 10 mm, the specified curvature threshold may be about two times the lesion radius, or up to five times the lesion radius to achieve a more thorough removal of the lesion. Device **100** may be adapted or selected according to the specific lesion that is to be treated therewith.

In case of airway obstruction having a radius between 0.5 mm and 5 mm for small obstructions and having a radius between 5 mm and 20 mm for large obstructions, the specified curvature threshold may be about two times the obstruction radius, or up to five times the obstruction radius to achieve a more thorough removal of the obstruction. Device **100** may be adapted or selected according to the specific obstruction that is to be treated therewith.

In case of gastrointestinal obstructions having a radius between 10 mm and **100** mm, the specified curvature threshold may be about two times the obstruction radius, or up to five times the obstruction radius to achieve a more thorough removal of the obstruction. Device **100** may be adapted or selected according to the specific obstruction that is to be treated therewith.

In case of obstructions in water or sewage pipes, having a radius between 50 mm and 500 mm, the specified curvature threshold may be about two times the obstruction radius, or up to five times the obstruction radius to achieve a more thorough removal of the obstruction. Device **100** may be adapted or selected according to the specific obstruction that is to be treated therewith.

Device **100** may be part of an arterial catheter system which includes a flexible elongate member or catheter with an outer surface, a distal region adapted to enter an artery and a proximal region extending from a patient's vessel, permitting control outside the patient's body by a physician. At the distal region of the catheter is the atherosclerosis treating assembly which may include suction and/or drug administration surface and a balloon expansion unit. Alternatively, in some embodiments it may include an atherectomy assembly which includes a cutting blade, abrasive member, snare etc. and a trapping mechanism which in certain embodiments comprises of openings in the trapping surface which extend to the proximal region of the catheter and are attached to a vacuum source. Alternatively, in some embodiments it may include an energy source like ultrasound, laser or RF.

The physician typically determines the presence and location of the plaque using one or several visualization techniques. The distal end of the arterial catheter is inserted and deployed through an incision in the femoral or brachial artery in a manner widely used in coronary and other arteries angioplasty, atherectomy and ultrasonography catheters. The catheter's distal region is advanced within the femoral or brachial artery until the distal end reaches the region of interest. Advancement of the catheter may be facilitated by X-ray fluoroscopy and the distal region of the catheter may include one or more fluoroscopic markers to enable such visualization. Advancement may also be facilitated by IVUS, TEE or by conventional guidewire and/or guiding catheter. In some cases, a distal protection unit or other filtration means will be used, either as part of the device or as a separate tool.

Once filtration/distal-protection and general lesion identification are established, the precise lesion identification mechanism is deployed. The precise lesion identification mechanism then enables the simple delivery of treatment via drug-administration, suction and/or one of several available energy sources.

**Figures 2A-2D** schematically illustrate variations of treatment application by operable elements **130**. For example, operable elements **130** may comprise openings that are configured to be opened mechanically by the bending of flexible supporting layer **120** beyond the specified curvature threshold. Through the openings, drugs **135** may be eluted (**Figure 2B**) or suction **136** may be applied to lesion **95**. Drug elution or suction may be applied through a cavity **125** that is additional to flexible supporting layer **120** or within flexible supporting layer **120**. As illustrated in **Figures 2B** and **2C** respectively, device **100** may comprise a drug delivery layer **125** or a suction layer **126** in fluid communication with the openings. In embodiments (**Figure 2D**), flexible supporting layer **120** itself may comprise a drug delivery volume or a suction volume in fluid communication with the openings.

The precise obstruction identification by the bending of flexible supporting layer **120** upon contact of treatment layer **110** with obstruction **95** may be facilitated by a compliant balloon **122**. Balloon**122** may be inflated such that its surface takes the shape of obstruction **95**. Balloon **122** may be inflated in a manner that permits blood flow while inflated. The balloon's surface may enable the administration of treatment by mechanical, electrical, chemical or other means only at the areas made concave due to the occlusion, as illustrated in the following examples.

During the operation of device **100**, lesions **95** such as plaque is reduced by suction, mechanical wear, laser, RF radiation, ultrasound or drug administration. The removal of lesion material results in a reduction of surface concavity to a level in which the bending of flexible supporting layer **120** is reduced, e.g. below the specified curvature threshold. As a result, treatment of lesion **95** may be stopped and concluded. Alternatively or additionally, the occlusion status may be monitored by sensing a status of operative elements **130** (e.g. the size of openings, the position of perforators etc. as explained below) or by detecting radio-opaque marking on device **100** the outline of a radio-opaque balloon supporting device **100**. When the treatment of lesion **95** (e.g. of an occlusion) is completed, device **100** is removed, e.g. by deflation of balloon **122**.

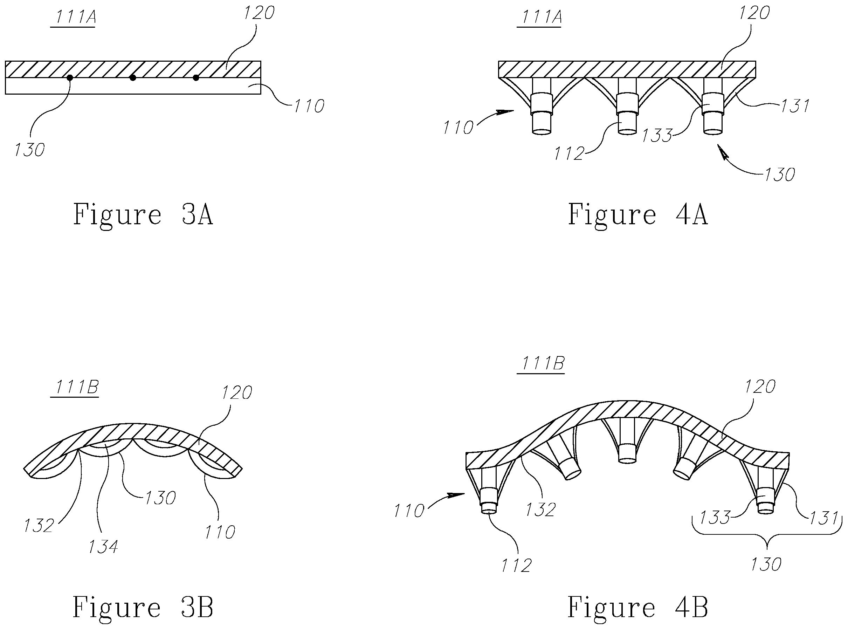

**Figures 3A** and **3B** are high level schematic illustrations of device **100** configuration having a non-active state **111A** and a bending-activated state **111B**, respectively, according to some embodiments of the invention. In state **111B**, treatment layer **110** is connected to flexible supporting layer **120** only at specified connection areas **132**, leaving the rest of treatment layer **110**, including operable elements **130**, to be separated from flexible supporting layer **120** upon inwards bending thereof. As a result, spaces **134** are formed between treatment layer **110** and flexible supporting layer **120**, which allow application of a treatment (e.g. drug elution) through operable elements **130**.

**Figures 4A** and **4B** are high level schematic illustrations of device **100** for perforating lesion **95**, having non-active state **111A** and bending-activated state **111B**, respectively, according to some embodiments of the invention. Operable elements **130** may comprise perforators **133** configured to penetrate vascular lesion **95** upon bending of flexible supporting layer **120** beyond the specified curvature threshold. The perforation may be the sole treatment of lesion **95** or it may instrumental for delivering drugs to lesion **95**, e.g. through openings in treatment layer **110** as described above and below, or for applying suction to fluids in lesion **95** via suction **136** as described above.

In the illustrated example, perforators **133** may be connected by supports **131** to connecting areas **132** on flexible supporting layer **120**. Upon bending flexible supporting layer **120**, perforators **133** may extend into lesion **95**, guided by supports **131**. Perforators may be hollow and extend distally to openings in flexible supporting layer **120**, in drug delivery layer **125** or in suction layer **126** and function as miniature needles for drug delivery or suction. Alternatively, perforators **133** may be mounted on guides **112** for stabilization and protection in non-operative state **111A**. Perforators **133** may then slide along guides **112** and extend beyond them in operative state **111B**. In embodiments, guides **112** may be hollow and deliver drugs or apply suction by connection to openings in flexible supporting layer **120**, in drug delivery layer **125** or in suction layer **126**.

In embodiments, perforators **133** may be extended into lesion **95** by mechanical or electrical means which cause perforators **133** to penetrate the artery's inner surface to enable easy access to lesion **95**. As the bending of flexible supporting layer **120** occurs only upon contact with lesion **95**, all other potential administration areas remain closed and do not affected the artery's surface during the procedure. This basically serves as an "automated" procedure in the sense that it only affects the occluded areas. The holes made by perforators **133** on the surface of lesion **95** may serve as openings for drug administration, suction or mechanical mining devices designated for the treated area mainly. Referring to **Figure 4B**, in the case of the mechanical apparatus, the surface's concave structure covering balloon **122** may be twisted due to side leverage by supports **131**. Referring to **Figure 3B**, a sliding or a radial movement between flexible supporting layer **120** and treatment layer **110** may be generated due to the varying radii of curvature of the layers. Either movement may be used, by appropriate configuration of device **100**, to facilitate an opening through treatment layer **110** for application of the various treatment methods. An electrical apparatus may also be used to perforate lesion **95**, by using curvature sensitive wiring to sense the concave surface of device **100** and electrically or mechanically open an opening in treatment layer **110** to access the plaque or perforate lesion **95**. In embodiments, drugs **135** may be eluted or suction **136** may be applied through perforators **133**.

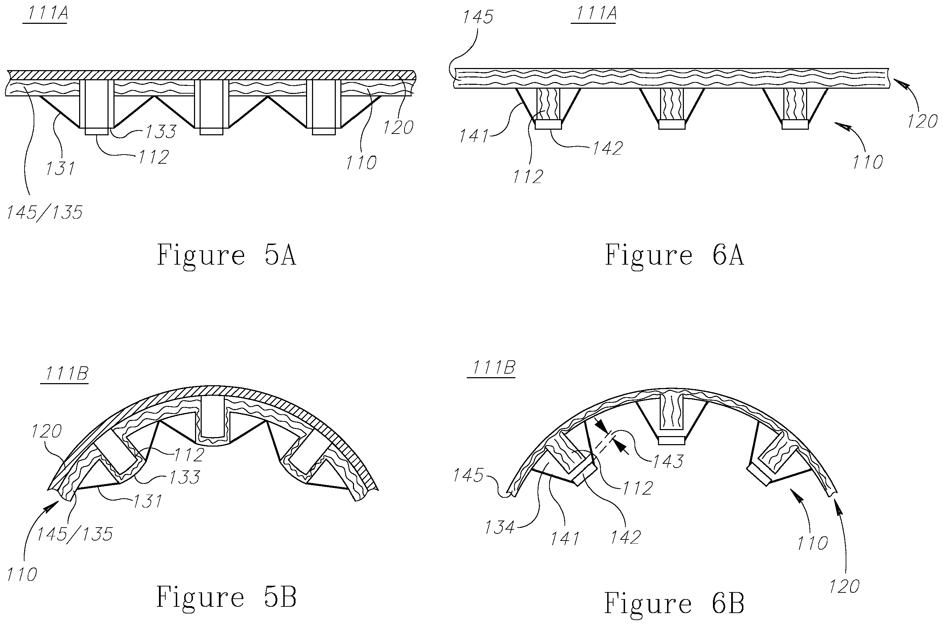

**Figures 5A** and **5B** are high level schematic illustrations of device **100** for perforating obstruction **95**, having non-active state **111A** and bending-activated state **111B**, respectively, and including an actuator **145**, according to some embodiments of the invention. Actuator **145** is arranged to control mechanically the bending of device **100** and thereby to control mechanically the operation of operable elements **130**. In this example, actuator **145** controls the extent to which perforators **133** are exposed (by withdrawal of tubes **112** due to the bending) and hence the depth of perforation applied to obstruction **95**.

Similar actuators **145** may be incorporated in other embodiments of the invention to add a control to the extent of bending experienced by device **100** and the operation of operable elements **130**. For example, actuator **145** may control the extent to which openings (see below) are opened, drugs are eluted etc. Actuator **145** may be drugs **135** themselves, that may be pressurized to control the activation of operable elements **130**. Actuator **145** may incorporate sensors for the bending of device **100** that may be used to monitor the treatment. In case drugs **135** are eluted in activated state **111B**, drugs **135** may be stored in non-activated state **111A**, e.g. within treatment layer, instead or in addition to actuator **145** as illustrated in **Figure 5A**. Alternatively, drugs **135** may be stored in non-activated state **111A** in supporting layer **120** as illustrated below.

Actuator **145** may be part of treatment layer **110**, of flexible supporting layer **120**, or be implemented as an additional layer of device **100** at any location with respect to layers **120** and **110**. Actuator **145** may be implemented as an elastic or a rigid wire, may be made of the same material as tubes **112** to control the extent of perforation resiliently or be associated with supports **131**.

**Figures 6A** and **6B** are high level schematic illustrations of device **100** for delivering a drug to lesion **95**, having non-active state **111A** and bending-activated state **111B**, respectively, according to some embodiments of the invention. In the illustrated embodiment, operable elements **130** comprise openings that are mechanically controlled by the extent of bending of device **100**.

Treatment layer **110** comprises tubes **112** that are covered by caps **142** in non-active state **111A**. Caps **142** may be supported by supports **141** or may be connected to the edges of tubes **112** or to supporting later **120**. Upon bending flexible supporting layer **120**, caps **142** move to create a gap **143** with respect to tubes **112**. Gap **143** may operate as an opening for eluting drugs **135**. Drugs **135** may be delivered by flexible supporting layer **120**, drug delivery layer **125** or treatment layer **110**, the latter e.g. via spaces **134** (**Figure 6B**).

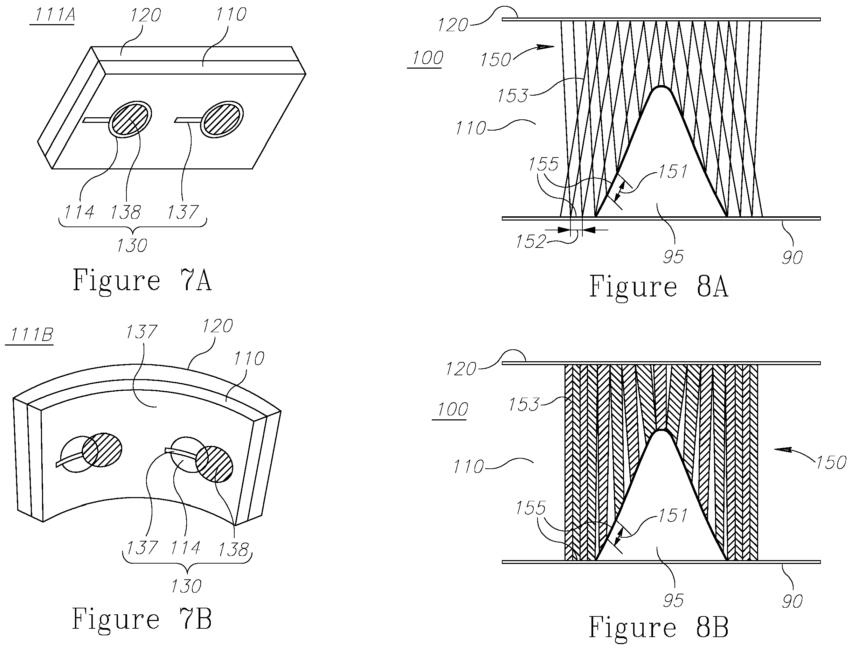

**Figures 7A** and **7B** are high level schematic illustrations of device **100** for delivering a drug to lesion **95**, having non-active state **111A** and bending-activated state **111B**, respectively, according to some embodiments of the invention. In the illustrated embodiment, operable elements **130** comprise openings **114** connected to flexible supporting layer **120**, drug delivery layer **125** or suction layer **126**, as explained above, and configured to elute drugs **135** or enable suction **136** there through.

In embodiments, operable elements **130** may comprise openings **114** that are covered by corresponding discs **138**, configured to expose at least one of openings **114** upon the bending of flexible supporting layer **120** beyond the specified curvature threshold. Each disc **138** may be connected to treatment layer **110** or to flexible supporting layer **120** by a holder **137**, that keeps disc **138** in place while opening **114** moves behind it due to the bending of flexible supporting layer **120**. Upon straightening of flexible supporting layer **120**, or in areas where flexible supporting layer **120** is not bend (e.g. as device **100** is not in contact with lesion **95**), discs **138** cover openings **114** and prevent drug elution or suction.

The density of openings **114** on the surface of obstruction **95** may vary according to the type of obstruction, type of treatment, type and size of openings, type of mechanical activation etc. A minimum of 5-10 openings **114** per obstruction is required. In non limiting examples, the following may be typical parameters for the density of openings **114** of different types, in cases of different obstructions.

In case of coronary lesions having a radius between 0.1 mm and 2 mm, the distance between adjacent openings **114** may be between 0.02 mm and 0.2 mm. In case of vascular lesions having a radius between 1 mm and 10 mm, the distance between adjacent openings **114** may be between 0.2 mm and 1 mm. In case of airway obstruction having a radius between 0.5 mm and 5 mm for small obstructions and having a radius between 5 mm and 20 mm for large obstructions, the distance between adjacent openings **114** may be between 0.1 mm and 0.5 mm for small obstructions and between 1 mm and 5 mm for large obstructions. In case of gastrointestinal obstructions having a radius between 10 mm and 100 mm, the distance between adjacent openings **114** may be between 2 mm and 10 mm. In case of obstructions in water or sewage pipes, having a radius between 50 mm and 500 mm, the distance between adjacent openings **114** may be between 0.1 mm and 0.5 mm In all these application cases, the density of openings **114** in device **100** and device **100** itself may be adapted or selected according to the specific obstruction or lesion that is to be treated therewith. Some of the applications involve continuous activation of operable elements **130** over a range of curvature radii (e.g. drug elution or suction), while other applications may involve activating the operable elements discretely (e.g. energy sources activation).

**Figures 8A** and **8B** are high level schematic illustrations of device **100** having a flexible network **150** with varying mesh openings **155**, according to some embodiments of the invention.

Treatment layer **110** may comprise flexible network **150** and have mesh openings **155** in network **150** as operable elements **130**. Network **150** is arranged to flex upon the bending to increase an opening size in a bended area of flexible network **150**. For example, network **150** may be compliant to the bending by lesion **95** and designed to widen its mesh openings **155** upon the network's compliance to the lesion's form.

In the illustrated example, mesh openings **155** in the bended regions are of a width **151** that is larger than a width **152** of mesh openings **155** in non-bended regions of network **150**. For example, width **151** may be three times width **152**. As illustrated in **Figure 8A**, network's wires **153** may be configured to be wide enough to prevent fluid communication through mesh openings **155** having width **152** but allow fluid communication through mesh openings **155** having width **151**. In another example, network wires **153** may have flaps of width **152** (not shown, the wires are shown schematically to illustrate mesh openings only), that are separated from each other only when the distance between wires **153** exceeds width **152** significantly, e.g. reach width **151**.

As illustrated in **Figure 8B**, wires **153** may be wide enough to leave no gap **155** between them at a low radius of curvature (left side of **Figure 8B**, on vessel **90**), but spread apart to expose gap **155** upon bending beyond the curvature threshold due to obstruction **95** (middle of **Figure 8B**, on lesion **95**).

In embodiments, the space within network **150** and/or treatment layer **110** may be used to elute drugs **135** or apply suction **136** to lesion **95**.

In embodiments, eluted drugs **135** may be photo activated to apply photodynamic therapy (PDT). Either or both drugs **135** and activating light may be delivered to lesion **95** via openings **114** or gap **155**.

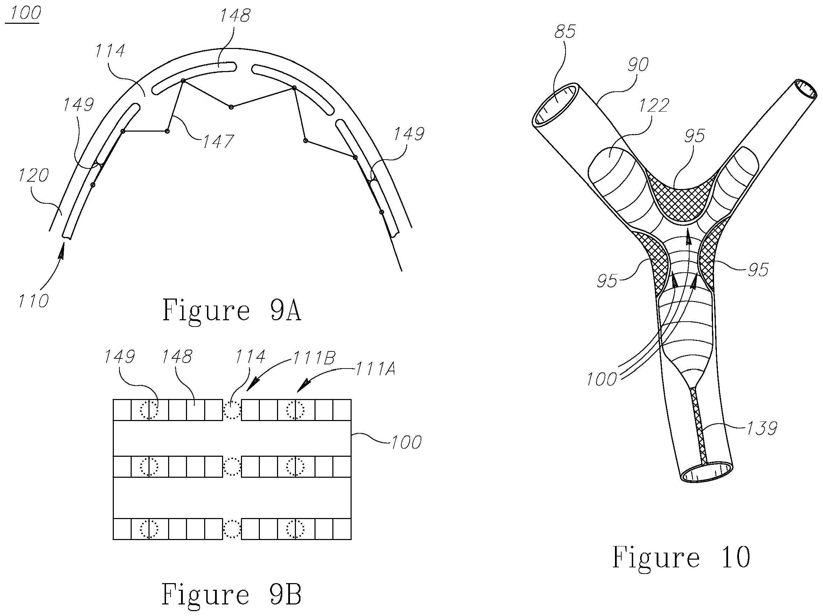

**Figures 9A** and **9B** are high level schematic illustrations of device **100** for delivering drug **135** to lesion **95**, according to some embodiments of the invention. In the illustrated embodiment, openings **114** in treatment layer **110** are operable elements **130**. treatment layer **110** may comprise multiple segments **148** which are held together by an expandable scaffold **147** that interconnects them and allow their separation upon bending of device **100** (**Figure 9A**). In a view from above, **Figure 9B** illustrates the interface between operable elements **130** and obstruction **95** - below the bending threshold segment **148** are close together and conceal closed openings **149**, while beyond bending threshold segment **148 are** spread apart to expose openings **114**.

**Figure 10** is a high level schematic illustration of balloon implemented device **100** for treating lesions **95** at vessel bifurcations, according to some embodiments of the invention.

Device **100** may be configured as balloon **122** (see also **Figure 2A** for a hollow balloon, allowing flow such as blood flow through cavity **85** in the balloon) with flexible supporting layer **130** being part of or externally attached to a skin of balloon **122**. In such embodiment, the bending of flexible supporting layer **120** is carried out by inflating balloon **122** against vascular lesion **95** and device **100** thus utilizes the lesion's natural curvature to activate operable elements **130**.

Balloon **122** may be connected to a delivery system **139** that is in fluid communication with drug delivery layer **125**, suction layer **126** or flexible supporting layer **120** to elute drugs **135** or apply suction **136**.

Furthermore, while vessel bifurcations are especially hard to treat with customary devices (because the prior art device must be shaped according to the bifurcation in advance), device **100** is particularly efficient is such cases as the curvature is enhanced by the bifurcation. In addition, device **100** is particularly efficient in treating the apex of the bifurcation, as the prior art disadvantage of the apex lesion having a small curvature radius become an advantage for the present invention, which utilizes the small curvature radius to enhance activation and treatment efficiency.

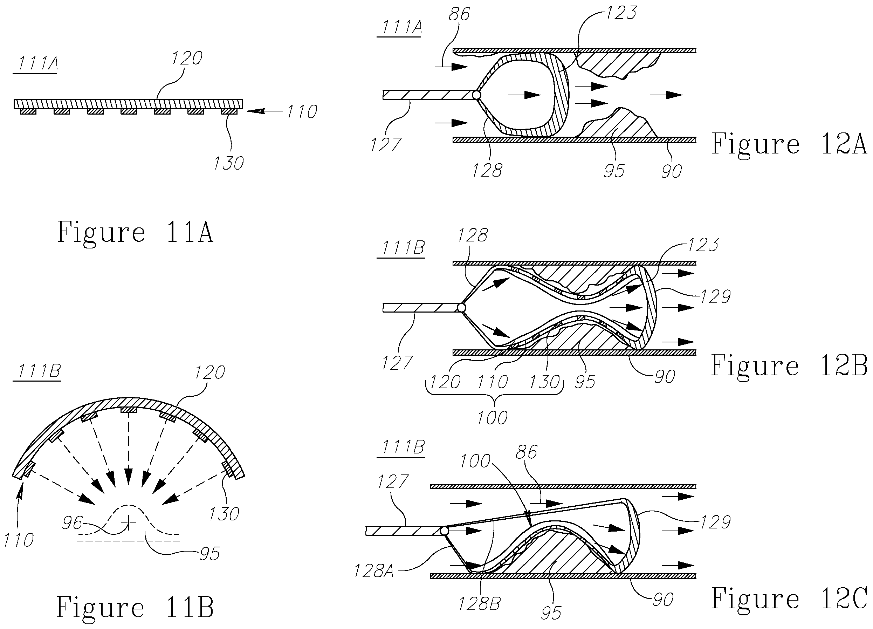

**Figures 11A** and **11B** are high level schematic illustrations of device **100** for focusing energy at obstruction **95**, having non-active state **111A** and bending-activated state **111B**, respectively, according to some embodiments of the invention.

In embodiments, operable elements **130** may comprise energy sources configured to deliver energy to vascular lesion **95**. For example, the energy sources may deliver electromagnetic radiation to obstruction **95** such as laser energy. Operable elements **130** may comprise the energy sources themselves or optical elements that are configured to direct and deliver the electromagnetic radiation from an external energy source (not shown) to obstruction **95**. In another example, the energy sources may deliver mechanical energy, e.g. in the form of ultrasound pressure waves, or electric energy, e.g. in the form of current. Operable elements **130** may comprise the energy sources themselves or transducers that receive energy from an external source (not shown).

The energy sources may be activated by the bending or may radiate at a low level that doesn't harm the surrounding. The effective energy level may be reached only upon adding several sources by the bending. Alternatively or complementary, the energy sources may be covered by treatment layer **110** and exposed to obstruction **95** upon widening of openings **114** in the cover, as described in other embodiments.

The concavity of device **100** may be used as a focusing mechanism of the energy sources. Curving treatment layer **110** over obstruction **95** points the energy sources to the same point **96** in the plaque body (in case of a vascular lesion), e.g. to the center of the plaque buildup.

Operable elements **130** may be positioned at such spaces and orientations that, upon the bending of flexible supporting layer **120** at a certain curvature, operable elements **130** deliver energy to specified focal areas **96** on obstruction **95**. In this way, energy from different energy sources is added and may be configured to be beyond a specified treatment threshold. In such a configuration, energy delivered from any single operable elements **130** may be selected to be harmless to surrounding tissue (in state **111A**), while upon concentration of several energy sources, due to the bending of device **100** on obstruction **95** (in state **111B**), an effective treatment is applied. In embodiments, operable elements **130** may comprise sensors that detect the bending of obstruction **95** and activate an external energy source (not shown) to deliver energy to operable elements **130** only upon detection of obstruction **95**.

**Figures 12A-12C** are high level schematic illustrations of a parachute implemented device **100** in stages of operation, according to some embodiments of the invention. The parachute form allows utilizing patterns of fluid flow **86** (e.g. blood flow through arteries) to deploy device **100** upon obstruction **95** by utilizing the flow distracting characteristics of obstruction **95**, in addition to utilizing the form of obstruction **95** for applying the treatment itself. Device **100** is configured to contact obstruction such as vascular lesion **95** by being carried along by blood flow **86** until being obstructed by vascular lesion **95**.

In embodiments, parachute **123** comprises a holder **127** connected via strings **128** to device **100** and configured to guide device **100** to bend upon obstruction **95** by utilizing fluid flow patterns in the vicinity of obstruction **95**. In embodiments, parachute **123** may further comprise a frontal leading area **129** configured to maintain a force resulting from fluid flow **86** and acting to position device **100** on obstruction **95**. In embodiments, leading area **129** may be part of device and may be used to apply treatment. Using parachute **123** further ensures maintaining fluid flow **86** through vessel **90** during the treatment, which may be an essential requirement, e.g. when treating lesions in the carotid arteries.

In embodiments, the parachute may be replaced by a torus shaped balloon (not shown) that allows blood flow therethrough. The operation of the torus shaped balloon may similarly utilize blood flow to place device **100** upon the lesion, or generally utilize fluid flow to place device **100** upon obstruction **95**.

**Figures 12B** and **12C** illustrate examples for deployment of parachute implemented device **100**, in a case of opposing lesions **95** or bilateral narrowing of vessel **90**, and in a case of a single lesion **95**, respectively. In the former case (**Figure 12B**), strings **128** may stabilize device **100** on both lesions **95** while frontal leading area **129** may ensure an appropriate coverage of lesions **95** by device **100**. In the latter case (**Figure 12C**), strings **128** may act asymmetrically to stabilize device **100** on lesion **95** while frontal leading area **129** may ensure an appropriate coverage of lesions **95** by device **100**. A string **128B** farther from lesion **95** may expand or be released to allow a far end of device drift further and cover lesion **95**, while a string **128A** closer to lesion **95** may shrink or be kept short to place a close end of device onto the beginning of lesion **95**. Such asymmetric operation utilizes fluid flow **86** to place device **100** onto lesion **95** and utilize its bending to apply the treatment.

Parachute **123** may be deployed by first releasing device **100** into the blood stream far enough from lesion **95**, such that flow is not affected by lesion **95**. Second, device **100** is deployed such that regions of device **100** that are in low flow or low flow resistance regions are anchored onto lesion **95** and regions of device **100** that are in high flow regions are not restricted. In this way device **100** tends to physically cover and deploy itself over the regions of lesion **95** which obstruct flow **86** the most. Parachute **123** may then be designed to include a therapeutic agent, an electric source, an ultrasound source or have light energy conducting properties to facilitate treatment by operable elements **130** at lesion **90** and prevent treatment or protect adjacent healthy regions. Furthermore, parachute **123** may be anchored to a catheter system for deployment and consequent retraction after treatment.

In embodiments, device **100** further comprises a flow sensor (not shown) for measuring the flow patterns near lesion **95**. The flow measurements may be used to plan the application of parachute implemented device **100**.

In embodiments, holder **127** may further comprise delivery system **139** in fluid communication with drug delivery layer **125**, suction layer **126** or flexible supporting layer **120** to elute drugs **135** or apply suction **136**.

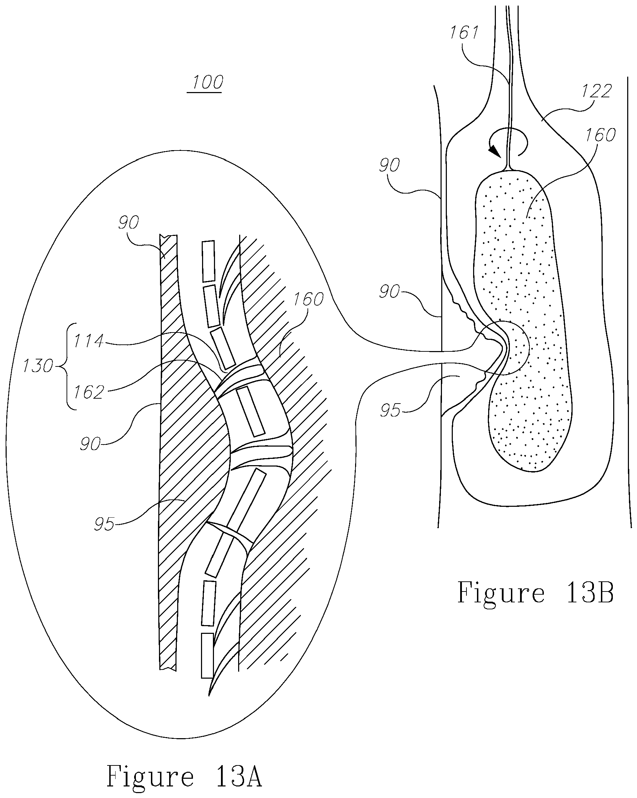

**Figures 13A** and **13B** are high level schematic illustrations of an obstruction grinding device **100**, according to some embodiments of the invention. Device **100** is designed to erode obstruction **95** through openings **114** in treatment layer **110**.

For example, a grinder **160** may be inserted into balloon **122** and rotated via handle **161**. Grinder **160** may have grinding protrusions **162** that may pass through openings **114** in activated state **111B** and erode obstruction **95**. Protrusions **162** may either be foldable in a deactivated state or spatially removed from layer **110** in inactive state **111A**.

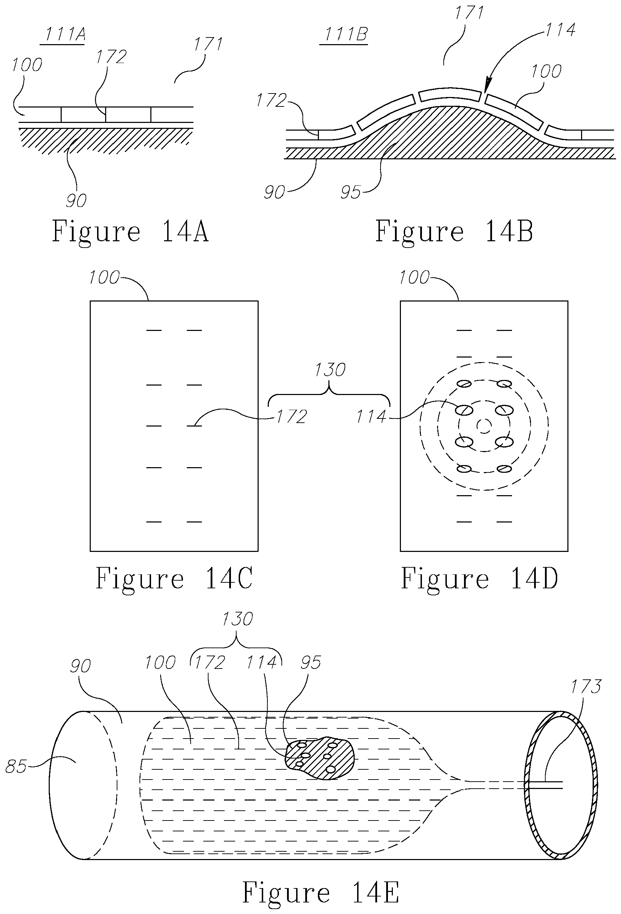

**Figures 14A-14E** are high level schematic illustrations of a one layered treatment device **100**, according to some embodiments of the invention. **Figures 14A-14E** illustrate in more detail an embodiment of device **100** depicted in **Figure 1C**, having openings **114** as operable elements. One layered treatment device **100** may be configured to be a part of a balloon skin, and activating openings **114** may create fluid communication between the balloon's interior **171** and obstruction **95**, that may be used for drug elution or application of suction, e.g. via channel **173**.

**Figures 14A**, **14C** and **14B**, **14D**, respectively, illustrate inactive state **111A** and activated state **111B**, the former with slits **172** that prevent fluid communication therethrough, and the latter with openings **114** resulting from the expansion of device **100** that turns slits **172** into openings **114** and provides fluid communication therethrough, for application of the treatment.

**Figure 14E** illustrates both states, the activated state over obstruction **95** and the inactive state over level areas of vessel **90**. The bending of device **100** over obstruction **95** yields the stretching and expansion of device **100** that turns slits **172** into openings **114** and provides fluid communication therethrough, for application of the treatment.

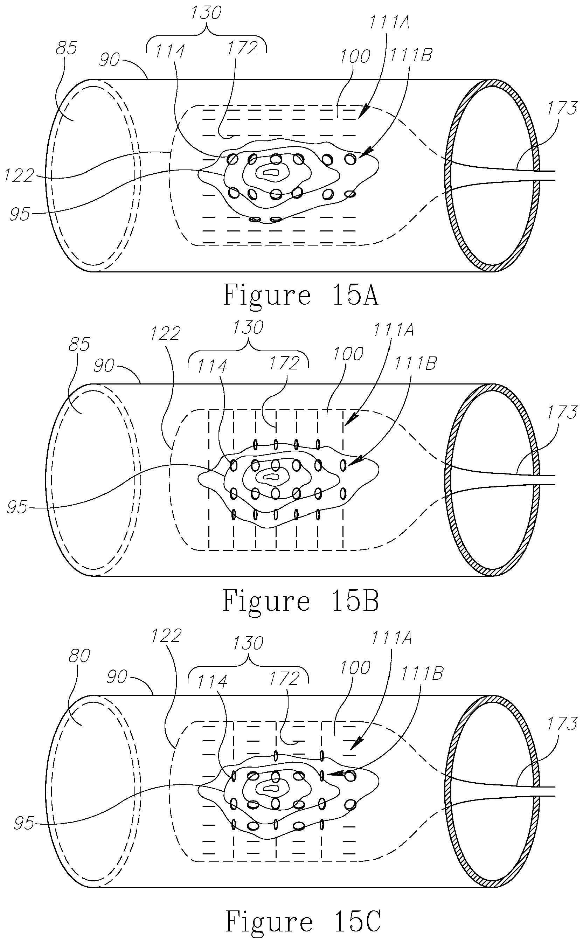

**Figures 15A-15C** are high level schematic illustrations of openings **114** as operable elements, according to some embodiments of the invention. **Figure 15A** illustrates a design of device **100** (one or two layered) with slits **172** and corresponding openings **114** along the direction of vessel **90** (and of flow **85** along vessel **90**). **Figure 15B** illustrates a design of device **100** (one or two layered) with slits **172** and corresponding openings **114** that are perpendicular to the direction of vessel **90** (and of flow **85** along vessel **90**). **Figure 15C** illustrates a design of device **100** (one or two layered) with a mixed configuration of slits **172** and corresponding openings **114**, some of slits **172** along the direction of vessel **90** and some perpendicular thereto.

The direction of slits **172** influences and allows spatial control of the mechanical activation of operable elements **130** (in the illustrated case openings **114**, but not limited thereto). In particular, the specified curvature threshold is defined with respect to slit direction - the radius of curvature of obstruction **95** must decrease below the given threshold curvature radius along a direction that is perpendicular to slit **172** to induce the maximal opening thereof. A direction of radius of curvature decrease that is tangential to the direction of slit **172** generates much less spreading of opening **114**, if at all (depending on the threshold values). Hence, the direction of slits **172** and openings **114** may be selected according to the local topography of obstruction **95** to apply the selected treatment. Any mixture of slits **172** having different orientations induces a different level of activation of operable elements with respect to a given obstruction topography, and hence allow a spatially differentiated treatment of obstruction **95**, i.e. certain regions in obstruction **95** may be treated at different intensities, or a given region may be treated at different intensities sequentially by different areas of device **100**.

In embodiments, the material of device **100** itself may be used to treat obstruction **95**. For example, device **100** may be implemented as balloon **122** or as the parachute itself and activate the treatment upon bending of device **100** as balloon or parachute skin upon obstruction **95**.

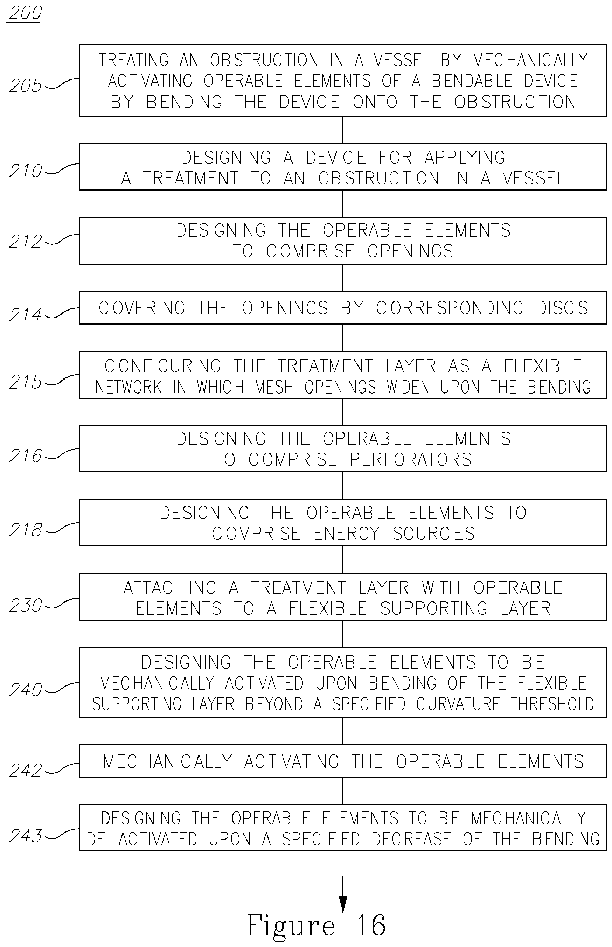

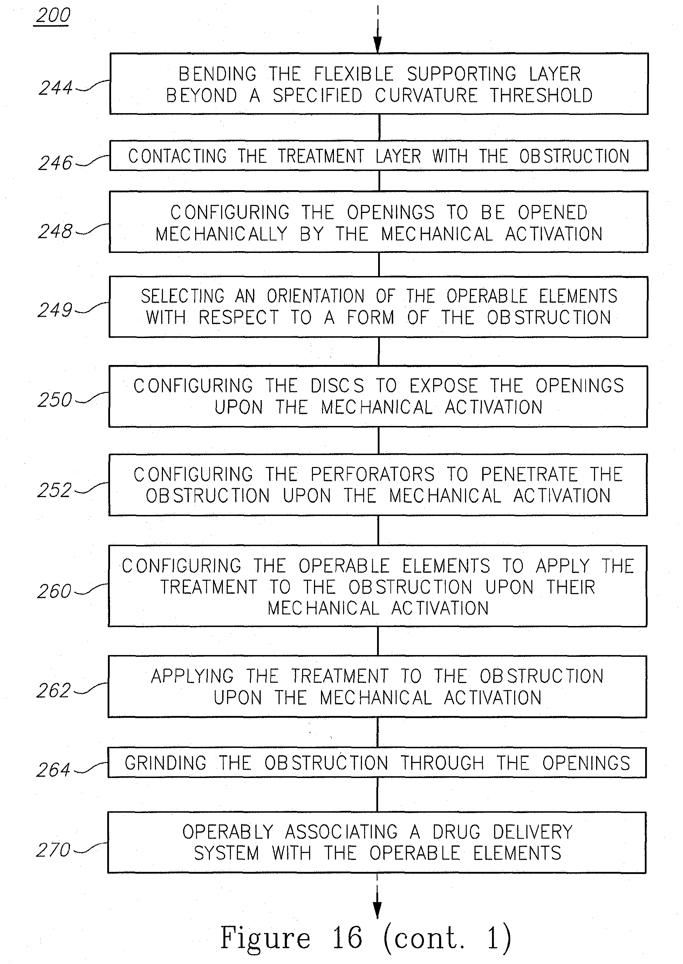

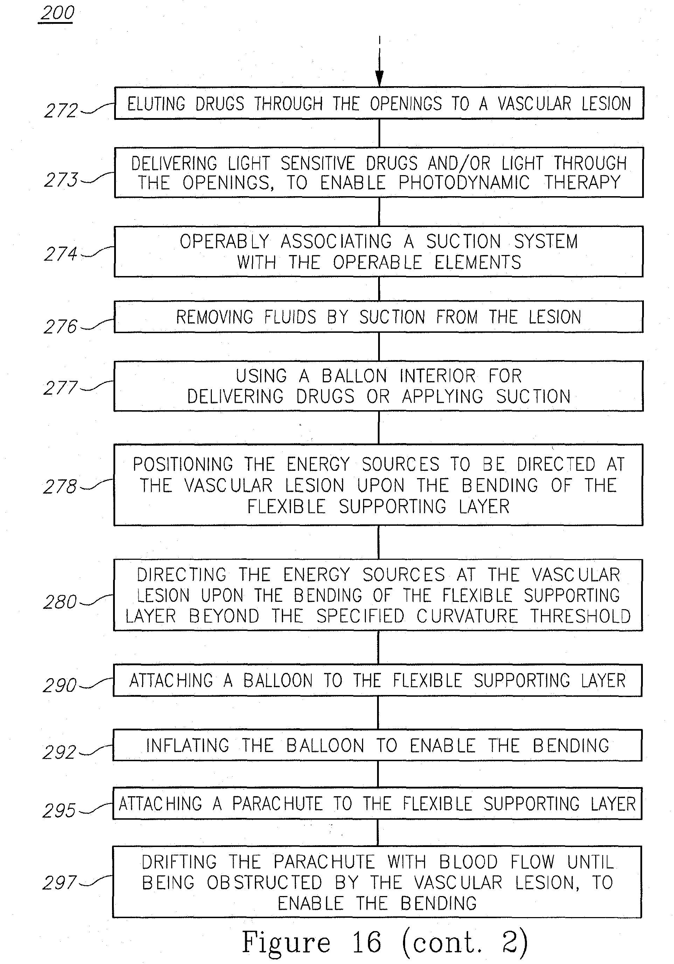

**Figure 16** is a high level flowchart illustrating an exemplary method **200** of designing a device for applying a treatment to an obstruction in a vessel.

Method **200** of designing a device for applying a treatment to an obstruction (stage **210**) comprises the following stages: attaching a treatment layer comprising a plurality of operable elements to a flexible supporting layer (stage **230**); designing (stage **240**) the operable elements to be mechanically activated (**242**) upon bending of the flexible supporting layer beyond a specified curvature threshold (**244**) due to contact of the treatment layer with the obstruction (such as a vascular lesion) (**246**); and configuring the operable elements (stage **260**) to apply the treatment (**262**) to the obstruction upon their mechanical activation.

Method **200** may further comprise designing the operable elements to be mechanically de-activated upon a specified decrease of the bending (stage **243**), due to the treatment of the obstruction.

Method **200** may further comprise designing the operable elements to comprise openings (stage **212**) and configuring the openings to be opened mechanically by the mechanical activation (stage **248**).

Method **200** may further comprise selecting an orientation of the operable elements with respect to a form of the obstruction (stage **249**) for example to apply a spatially differentiated treatment. Curvature thresholds may be selected according to the orientation of the operable elements or vice versa. Curvature thresholds may vary for different orientations on obstruction **95**.

Method **200** may further comprise covering the openings by corresponding discs (stage **214**), and configuring the discs to expose the openings upon the mechanical activation (stage **250**).

Method 200 may further comprise configuring the treatment layer as a flexible network in which mesh openings widen upon the bending (stage 215). The treatment may be applied utilizing the wider mesh openings to treat the lesion specifically.

Method 200 may further comprise designing the operable elements to comprise perforators (stage 216), and configuring the perforators to penetrate the obstruction (e.g. a vascular lesion) upon the mechanical activation (stage 252).

Method 200 may further comprise operably associating a drug delivery system with the operable elements (stage **270**) and eluting drugs to the lesion thereby (stage **272**). Method **200** may further comprise delivering light sensitive drugs and/or light through the openings, to enable photodynamic therapy (stage **273**).

Method **200** may further comprise operably associating a suction system with the operable elements (stage **274**) and removing fluids by suction from the lesion thereby (stage **276**).

Method **200** may further comprise designing the operable elements to comprise energy sources (**218**), and positioning the energy sources (stage **278**) to be directed (**280**) at the obstruction upon the bending of the flexible supporting layer beyond the specified curvature threshold (**244**).

Method **200** may further comprise attaching a balloon to the flexible supporting layer (stage **290**) to enable the bending (**244**) by inflating the balloon (stage **292**).

Method **200** may further comprise attaching a parachute to the flexible supporting layer (stage **295**) to enable the bending (**244**) by drifting the parachute with flow (e.g. blood flow) until being obstructed by the vascular lesion (stage **297**). In examples, method **200** comprises treating an obstruction (stage **205**) by mechanically activating operable elements of a bendable device (**242**) by bending the device onto the obstruction (**244**).

For example, treating a vascular lesion (stage **205**) comprises eluting drugs into the lesion (stage **272**) through openings, exposed (**248**) or perforated (**252**) by the operable elements. In another example, treating the vascular lesion (stage **205**) comprises removing fluids by suction from the lesion (stage **276**) through openings, exposed (**248**) or perforated (**252**) by the operable elements.

In examples, treating the vascular lesion (stage **205**) comprises directing the energy sources at the vascular lesion upon the bending of the flexible supporting layer beyond the specified curvature threshold (stage **280**).

Contacting the vascular lesion may be carried out by inflating a balloon attached (**290**) to the flexible supporting layer (stage **292**) or by drifting a parachute attached to the flexible supporting layer (**295**) with blood flow until being obstructed by the vascular lesion, to enable the bending (stage **297**).

In the above description, an embodiment is an example or implementation of the invention. The various appearances of "one embodiment", "an embodiment" or "some embodiments" do not necessarily all refer to the same embodiments.

The invention is not limited to those diagrams or to the corresponding descriptions. For example, flow need not move through each illustrated box or state, or in exactly the same order as illustrated and described.

Meanings of technical and scientific terms used herein are to be commonly understood as by one of ordinary skill in the art to which the invention belongs, unless otherwise defined.

While the invention has been described with respect to a limited number of embodiments, these should not be construed as limitations on the scope of the invention, but rather as exemplifications of some of the preferred embodiments. Other possible variations, modifications, and applications are also within the scope of the invention. Accordingly, the scope of the invention is limited only by the appended claims.

## Claims

1. A device for applying a treatment to an obstruction, the device comprising:
a flexible supporting layer; and
a treatment layer comprising a plurality of operable elements attached to the flexible supporting layer and are configured to be activated to apply the treatment to the obstruction upon bending of the flexible supporting layer beyond a specified curvature threshold due to contact of the treatment layer with the obstruction.

2. The device of claim 1, wherein the operable elements are further configured to be de-activated upon a specified decrease of the bending, due to the treatment of the obstruction.

3. The device of claim 1 or 2, wherein the operable elements comprise openings that are configured to be opened mechanically by the bending of the flexible supporting layer beyond the specified curvature threshold.

4. The device of claim 1 or 2, wherein the operable elements further comprise perforators configured to penetrate the obstruction upon bending of the flexible supporting layer beyond the specified curvature threshold.

5. The device of any one of claims 1-4, wherein the flexible supporting layer comprises a drug delivery volume in fluid communication with the openings.

6. The device of claim 1 or 2, wherein the operable elements are energy sources configured to deliver energy to the obstruction and positioned to be directed upon the bending of the flexible supporting layer beyond the specified curvature threshold at the obstruction.

7. The device of claim 1 or 2, wherein the operable elements are optical elements that are configured to direct and deliver electromagnetic radiation from an external energy source.

8. The device of any one of claims 1-7, configured as a balloon with the flexible supporting layer being part of or externally attached to a skin of the balloon, wherein the bending of the flexible supporting layer is carried out by inflating the balloon against the obstruction.

9. The device of any one of claims 1-7, configured to contact the obstruction by being carried along by flow until being obstructed by the obstruction.

10. The device according to any of claims 1 to 9, wherein the obstruction is a vascular lesion and the treatment layer is configured to treat the vascular lesion.

11. A method of providing a device for applying a treatment to an obstruction, the method comprising:
attaching a treatment layer comprising a plurality of operable elements to a flexible supporting layer;
configuring the operable elements to be activated upon bending of the flexible supporting layer beyond a specified curvature threshold due to contact of the treatment layer with the obstruction; and
configuring the operable elements to apply the treatment to the obstruction upon their activation.

12. The method of claim 11, further comprising configuring the operable elements to be de-activated upon a specified decrease of the bending, due to the treatment of the obstruction.

13. The method of claim 11 or 12, further comprising configuring the operable elements to be optical elements that are configured to direct and deliver electromagnetic radiation from an external energy source.

14. The method of claim 11 or 12, wherein the operable elements comprise energy sources, and further comprising positioning the energy sources to be directed at the obstruction upon the bending of the flexible supporting layer beyond the specified curvature threshold.

15. The method of any one of claims 11-14, further comprising configuring the treatment layer to deliver at least one of light sensitive drugs and light through the openings, to enable photodynamic therapy.

## Patentansprüche

1. Vorrichtung zum Anwenden einer Behandlung an einer Obstruktion, wobei die Vorrichtung Folgendes umfasst:
eine flexible Trägerschicht; und
eine Behandlungsschicht, die eine Vielzahl von betriebsfähigen Elementen umfasst, die an der flexiblen Trägerschicht angebracht und dazu konfiguriert sind, dass sie aktiviert werden können, um die Behandlung beim Biegen der flexiblen Trägerschicht über eine angegebene Biegungsschwelle hinaus aufgrund eines Kontakts der Behandlungsschicht mit der Obstruktion an der Obstruktion anzuwenden.

2. Vorrichtung nach Anspruch 1, wobei die betriebsfähigen Elemente weiter dazu konfiguriert sind, dass sie bei einer angegebenen Abnahme der Biegung aufgrund der Behandlung der Obstruktion deaktiviert werden können.

3. Vorrichtung nach Anspruch 1 oder 2, wobei die betriebsfähigen Elemente Öffnungen umfassen, die dazu konfiguriert sind, dass sie durch das Biegen der flexiblen Trägerschicht über die angegebene Biegungsschwelle hinaus mechanisch geöffnet werden können.

4. Vorrichtung nach Anspruch 1 oder 2, wobei die betriebsfähigen Elemente weiter Perforatoren umfassen, die dazu konfiguriert sind, dass sie beim Biegen der flexiblen Trägerschicht über die angegebene Biegungsschwelle hinaus die Obstruktion durchdringen.

5. Vorrichtung nach einem der Ansprüche 1-4, wobei die flexible Trägerschicht ein Arzneistoffabgabevolumen in Fluidverbindung mit den Öffnungen umfasst.

6. Vorrichtung nach Anspruch 1 oder 2, wobei die betriebsfähigen Elemente Energiequellen sind, die dazu konfiguriert sind, dass sie Energie an die Obstruktion abgeben, und dazu positioniert sind, dass sie beim Biegen der flexiblen Trägerschicht über die angegebene Biegungsschwelle hinaus auf die Obstruktion gerichtet werden können.

7. Vorrichtung nach Anspruch 1 oder 2, wobei die betriebsfähigen Elemente optische Elemente sind, die dazu konfiguriert sind, dass sie elektromagnetische Strahlung von einer externen Energiequelle lenken und abgeben.

8. Vorrichtung nach einem der Ansprüche 1-7, die als Ballon konfiguriert ist, wobei die flexible Trägerschicht Teil einer Haut des Ballons oder außen an dieser befestigt ist, wobei das Biegen der flexiblen Trägerschicht durch Aufblasen des Ballons gegen die Obstruktion durchgeführt wird.

9. Vorrichtung nach einem der Ansprüche 1-7, die dazu konfiguriert ist, dass sie mit der Obstruktion in Kontakt tritt, indem sie von einer Strömung mitgeführt wird, bis sie durch die Obstruktion obstruiert wird.

10. Vorrichtung nach einem der Ansprüche 1 bis 9, wobei die Obstruktion eine vaskuläre Läsion ist und die Behandlungsschicht dazu konfiguriert ist, dass sie die vaskuläre Läsion behandelt.

11. Verfahren zum Bereitstellen einer Vorrichtung zum Anwenden einer Behandlung an einer Obstruktion, wobei das Verfahren Folgendes umfasst:
Anbringen einer Behandlungsschicht, die eine Vielzahl von betriebsfähigen Elementen umfasst, an eine flexible Trägerschicht;
Konfigurieren der betriebsfähigen Elemente, dass sie beim Biegen der flexiblen Trägerschicht über eine angegebene Biegungsschwelle hinaus aufgrund eines Kontakts der Behandlungsschicht mit der Obstruktion aktiviert werden können; und
Konfigurieren der betriebsfähigen Elemente, dass sie bei ihrer Aktivierung die Behandlung an der Obstruktion anwenden.

12. Verfahren nach Anspruch 11, weiter umfassend Konfigurieren der betriebsfähigen Elemente dazu, dass sie bei einer angegebenen Abnahme der Biegung aufgrund der Behandlung der Obstruktion deaktiviert werden können.

13. Verfahren nach Anspruch 11 oder 12, weiter umfassend Konfigurieren der betriebsfähigen Elemente dazu, dass sie optische Elemente sind, die dazu konfiguriert sind, dass sie elektromagnetische Strahlung von einer externen Energiequelle lenken und abgeben.

14. Verfahren nach Anspruch 11 oder 12, wobei die betriebsfähigen Elemente Energiequellen umfassen und weiter umfassend Positionieren der Energiequellen, dass sie beim Biegen der flexiblen Trägerschicht über die angegebene Biegungsschwelle hinaus auf die Obstruktion gerichtet werden können.

15. Verfahren nach einem der Ansprüche 11-14, weiter umfassend Konfigurieren der Behandlungsschicht dazu, dass sie mindestens eins von lichtempfindlichen Arzneistoffen und Licht durch die Öffnungen abgibt, um fotodynamische Therapie zu ermöglichen.

## Revendications

1. Dispositif permettant d'appliquer un traitement à une obstruction, le dispositif comprenant :
une couche de support flexible ; et
une couche de traitement comprenant une pluralité d'éléments fonctionnels fixés à la couche de support flexible et qui sont configurés pour être activés pour appliquer le traitement à l'obstruction lors d'une flexion de la couche de support flexible au-delà d'un seuil de courbure spécifié, du fait d'un contact de la couche de traitement avec l'obstruction.

2. Dispositif selon la revendication 1, dans lequel les éléments fonctionnels sont en outre configurés pour être désactivés lors d'une diminution spécifiée de flexion, du fait du traitement de l'obstruction.

3. Dispositif selon la revendication 1 ou 2, dans lequel les éléments fonctionnels comprennent des ouvertures qui sont configurées pour être mécaniquement ouvertes par la flexion de la couche de support flexible au-delà du seuil de courbure spécifié.

4. Dispositif selon la revendication 1 ou 2, dans lequel les éléments fonctionnels comprennent en outre des perforateurs configurés pour pénétrer l'obstruction lors d'une flexion de la couche de support flexible au-delà du seuil de courbure spécifié.

5. Dispositif selon l'une quelconque des revendications 1 à 4, dans lequel la couche de support flexible comprend un volume d'administration de médicament en communication fluidique avec les ouvertures.

6. Dispositif selon la revendication 1 ou 2, dans lequel les éléments fonctionnels sont des sources d'énergie configurées pour administrer de l'énergie à l'obstruction et positionnées pour être dirigées, lors de la flexion de la couche de support flexible au-delà du seuil de courbure spécifié, au niveau de l'obstruction.

7. Dispositif selon la revendication 1 ou 2, dans lequel les éléments fonctionnels sont des éléments optiques qui sont configurés pour diriger et émettre un rayonnement électromagnétique à partir d'une source d'énergie externe.

8. Dispositif selon l'une quelconque des revendications 1 à 7, configuré en tant que ballonnet à l'extérieur d'une peau duquel la couche de support flexible fait partie ou est fixée, dans lequel la flexion de la couche de support flexible est effectuée en gonflant le ballonnet contre l'obstruction.

9. Dispositif selon l'une quelconque des revendications 1 à 7, configuré pour venir en contact avec l'obstruction en étant entraîné par écoulement jusqu'à ce qu'il soit bloqué par l'obstruction.

10. Dispositif selon l'une quelconque des revendications 1 à 9, dans lequel l'obstruction est une lésion vasculaire et la couche de traitement est configurée pour traiter la lésion vasculaire.

11. Procédé de fourniture d'un dispositif permettant d'appliquer un traitement à une obstruction, le procédé comprenant :
la fixation d'une couche de traitement comprenant une pluralité d'éléments fonctionnels à une couche de support flexible ;
la configuration des éléments fonctionnels pour qu'ils soient activés lors d'une flexion de la couche de support flexible au-delà d'un seuil de courbure spécifié, du fait du contact de la couche de traitement avec l'obstruction ; et
la configuration des éléments fonctionnels pour appliquer le traitement à l'obstruction lors de leur activation.

12. Procédé selon la revendication 11, comprenant en outre la configuration des éléments fonctionnels pour qu'ils soient désactivés lors d'une diminution spécifiée de la flexion, du fait du traitement de l'obstruction.

13. Procédé selon la revendication 11 ou 12, comprenant en outre la configuration des éléments fonctionnels pour qu'ils soient des éléments optiques qui sont configurés pour diriger et émettre un rayonnement électromagnétique à partir d'une source d'énergie externe.

14. Procédé selon la revendication 11 ou 12, dans lequel les éléments fonctionnels comprennent des sources d'énergie, et comprenant en outre le positionnement des sources d'énergie pour qu'elles soient dirigées vers l'obstruction lors de la flexion de la couche de support flexible au-delà du seuil de courbure spécifié.

15. Procédé selon l'une quelconque des revendications 11 à 14, comprenant en outre la configuration de la couche de traitement pour distribuer au moins l'un parmi des médicaments photosensibles et de la lumière à travers les ouvertures, pour permettre une thérapie photodynamique.
